# EUROPEAN PATENT SPECIFICATION

(11) **EP 1 185 282 B1**
(45) Date of publication and mention of the grant of the patent: **12.03.2003**
(21) Application number: 00937016.4
(22) Date of filing: 01.06.2000
(51) Int. Cl.: A61K 35/78, A23L 1/30, A23L 1/305, A61P 13/10

(54) **FORMULATIONS CONTAINING CRANBERRY FRUIT, DL-METHIONINE, AND CHINESE HERBS**
ZUBEREITUNGEN, WELCHE PREISELBEEREN-FRUCHT, DL-METHIONIN UND CHINESISCHE KRÄUTER ENTHALTEN
FORMULATIONS CONTENANT DE L'AIRELLE, DE LA DL-METHIONINE ET DES HERBES MEDICINALES CHINOISES

(30) Priority: 02.06.1999 US 137196 P; 14.09.1999 US 153817 P
(43) Date of publication of application: 13.03.2002
(73) Proprietor: Oxford Natural Products PLC, Oxfordshire OX7 3EH (GB)
(72) Inventor: YU, Hongwen, Oxford OX1 3JS (GB); ZHONG, Shouming, Oxford OX1 3JS (GB); PACIORETTY, Linda, Brooktondale, NY 14817 (US); BABISH, John, G., Brooktondale, NY 14817 (US)
(74) Representative: Keen, Celia Mary
(86) International application number: GB0002094
(87) International publication number: WO00074697

(56) References cited:
- WO-A-95/26197
- WO-A-99/12541
- GB-A- 2 106 387
- US-A- 5 773 004
- DATABASE WPI Section Ch, Week 199204 Derwent Publications Ltd., London, GB; Class D13, AN 1992-027320 XP002146692 & JP 03 272652 A (NISSHIN FLOUR MILLING CO), 4 December 1991 (1991-12-04)
- KUBO T ET AL: "STUDY OF THE CONSTITUENTS OF DESMODIUM-STYRACIFOLIUM" CHEMICAL & PHARMACEUTICAL BULLETIN (TOKYO), vol. 37, no. 8, 1989, pages 2229-2231, XP002146691 ISSN: 0009-2363

## Description

The present invention relates to herbal formulations comprising cranberry fruit and Chinese herbs which can be administered as dietary supplements or as pharmaceutical formulations to prevent or treat the symptoms of urinary cystitis.

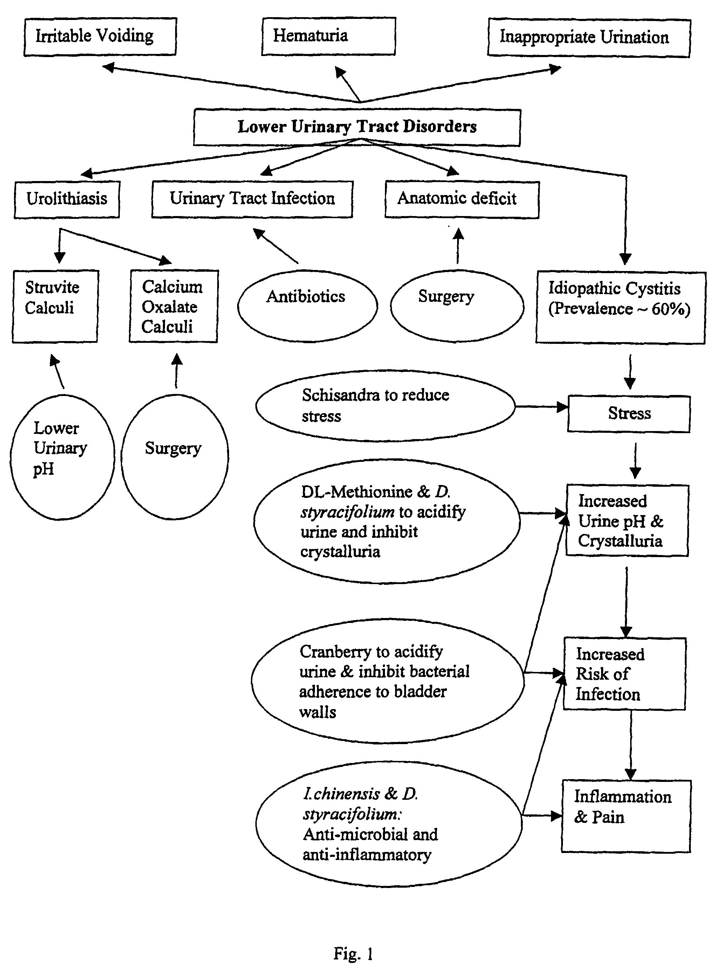

Recurring cystitis or urologic syndrome generally manifests a combination of symptoms including iritative voiding, hematuria or inappropriate urination. Often seen in females of nearly all species, the most common form is termed idiopathic lower urinary tract disease or idiopathic cystitis. Other forms ofurologic syndrome include urolithiasis, urinary tract infections and, least common, anatomic deficits (Figure 1).

There appears to be no single, primary causative agent associated with idiopathic cystitis. Among the risk factors identified, stress and aurinary pH greater than 7.0 are most frequently reported. While bacterial infection does not seem to play a primary role in the disease, several research groups have proposed that viral infections may have a predisposing influence. Low fluid intake is also considered a significant risk factor for recurrent idiopathic cystitis.

Antibiotics, steroids, diet change, and/or increased water intake are the commonly prescribed treatments. But treatment of idiopathic cystitis with presently available formulations has drawbacks. For example, reduction of stress is considered critical to terminating the cycle of recurrence. Treatment with amitriptyline, one of the early tricyclic antidepressants, has been reported to be highly effective in reducing the frequency of episodes in severe recurrent cases of idiopathic cystitis. However, potential side-effects of amitriptyline include sedation, hypotension, and weight gain.

Moreover, episodes of idiopathic cystitis tend to be self-limiting and resolve within ten days regardless of the treatment, but if the risk factors for idiopathic cystitis are not addressed, the likelihood of recurrence is high. A frequent complication in active untreated cystitis includes increased risk of infection, often leading to inflammation and painful urination.

Research suggests that acidifying the urine may be effective in the management of recurrent idiopathic cystitis. Ingestion of cranberry fruit juice and DL-methionine are speculated to help provide such acidification. One double-blind, placebo-controlled study of 153 elderly women indicated that the consumption of 300 ml of commercially available standard cranberry juice reduced the odds of bacteriuria by forty-two percent. Avorn et al., *Reduction of bacteriuria and pyuria after ingestion of cranberry juice*, 271 JAMA 751-754 (1994). Other studies suggest that cranberry juice may be more effective in treating than in preventing bacteriuria and urinary tract infections. Fleet, J.C. *New support for a folk remedy: cranberry juice reduces bacteriuria and pyuria in elderly women*, 52 NUTR. REV.168-170 (1994). However, it is unclear whether the positive results obtained with cranberry fruit juice are due to some ingredient of cranberries, such as quinic acid which is thought to have an antimicrobial role, or whether any observed positive results are due simply to the consumption of additional fluids.

While ingestion of cranberry fruit juice is thought to be useful for alleviating urinary - cystitis, any positive clinical results obtained with cranberry fruit juice may simply be due to the consumption of additional fluids, a practice known to reduce the risk of urinary tract infections. Furthermore, even if historical and clinical use of cranberry fruit juice indicates that cranberries are useful for treating urinary tract infections, cranberry fruit extract has not been optimally formulated into a dietary supplement or a pharmaceutical composition that can effectively relieve all the symptoms of recurrent idiopathic cystitis.

Accordingly the present invention provides a herbal composition comprising cranberry fruit, DL-methionine and at least one herb selected from *Ilex chinensis, Desmodium styracifolium* and *Schisandra chinensis*. In a preferred aspect the herbal composition comprises cranberry fruit, DL-methionine and each of *Ilex chinensis, Desmodium styracifolium* and *Schisandra chinensis*.

In a more preferred aspect the herbal composition comprises synergistically effective amounts of the cranberry fruit, DL-methionine and the or each said herb. A Chinese herb selected from *Ilex chinensis, Desmodium styracifolium* and *Schisandra chinensis* may thus be used as a synergist in a herbal composition comprising cranberry fruit and DL-methionine. Such a herbal composition preferably further comprises one or both of the other two said Chinese herbs. Thus *Ilex chinensis* may be used as a synergist in a herbal composition comprising cranberry fruit, DL-methionine and one or both of *Desmodium styracifolium* and *Schisandra chinensis*; *Desmodium styracifolium* may be used as a synergist in a herbal composition comprising cranberry fruit, DL-methionine and one or both of *Ilex chinensis* and *Schisandra chinensis;* and *Schisandra chinensis* may be used as a synergist in a herbal composition comprising cranberry fruit, DL-methionine and one or both of *Ilex chinensis* and *Desmodium styracifolium*.

DL-methionine is an amino acid which may help acidify urine and is available commercially. DL-Methionine has been used clinically to acidify the urine. Doses of 1500 to 3000 mg/day in humans reduced the mean pH values of the urine of 19 subjects from 7.5 to 5.5 (p<0.05). Jarrar, K., R.H. et al., *Struvite stones: long term follow-up under metaphylaxis*, 30 ANN UROL. (Paris)112-117(1996).

Some information indicates that an herbal extract of *Ilex chinensis* is both an antimicrobial and an antiinflammatory agent. This herb has also been reported to reduce vascular resistance in coronary vessels resulting in an increase in blood flow. Following oral administration, the herb is almost completely absorbed from the small intestine and eliminated primarily through the kidneys. The antibacterial effect of *Ilex chinensis* allows it to be effective in the treatment of infectious diseases such as acute and chronic bronchitis, pneumonia, dysentery, nephritis, pelvic infections, urethritis and cervicitis.

Information also exists indicating that herbal extracts of *Desmodium styracifolium* may have anti-inflammatory activity. *Desmodium styracifolium* has been found to contain several classes of biologically active compounds. The flavones and triterpenoids identified in *Desmodium styracifolium* may be responsible for the anti-inflammatory activity ascribed to the herb. In rat studies, the triterpenoid fraction inhibited the formation of experimentally-induced calcium oxalate renal stones.

Schisandra fruit, derived from *Schisandra chinensis,* has been used in China as both a foodstuff and a medicinal herb. It has been classified as an adaptogen because of its ability to balance and regulate many functions of the body.

Theherbal composition of the invention, comprising cranberry fruit, DL-methionine and one or more of the herbs as described above, is an ideal formulation for the reduction of risk factors associated with cystitis since it (i) decreases urinary pH, (ii) reduces physiological stress, (iii) reduces pain, and (iv) decreases the inflammatory response. It therefore adds to the potential acidification properties offered by cranberry fruit by addressing the additional risk factors for cystitis of physiological stress, inflammation and infection. Working together the components of the composition reduce the frequency of recurrent idiopathic cystitis and normalize. urinary tract function. Furthermore, the composition is inexpensively manufactured and complies with all governmental regulations.

The present invention also provides a pharmaceutical or veterinary composition comprising a herbal composition of the invention as defined above and a pharmaceutically or veterinarily acceptable excipient.

The present invention further provides a dietary composition which comprises a herbal composition of the invention as defined above and a dietetically acceptable excipient. The dietary composition may be a dietary supplement.

The herbal composition of the present invention may be used in a method of dietary supplementation which comprises administration of the composition to a human or animal in an amount sufficient to support healthy urinary tract function. This method can thus be used to enhance the urinary health of mammals.

The invention further provides the use of cranberry fruit, DL-methionine and at least one Chinese herb selected from *Ilex chinensis, Desmodium styracifolium* and *Schisandra chinensis* in the manufacture of a medicament for preventing or treating one or more symptoms of urinary cystitis. In one embodiment of the invention the DL-methionine and the or each said Chinese herb are used in synergistically effective amounts.

The herbal composition of the present invention as defined above may be used in a method of treating urinary cystitis, for instance recurrent idiopathic cystitis, which comprises the administration to a human or animal patient in need thereof a pharmaceutical or veterinary composition comprising the herbal composition of the invention and continuing the administration of the formulation until the symptoms are reduced. Preferably the composition comprises a synergistically effective amount of cranberry extract, DL-methionine, *Ilex chinensis, Desmodium styracifolium,* and *Schisandra chinensis*.

The symptoms of cystitis to be treated include any abnormal urinary function, irritative voiding, hematuria, inappropriate urination, urolithiasis or urinary tract infection..

In the accompanying drawing, Figure 1 provides a schematic of the breakdown of lower urinary tract disorders and the risk factors associated with idiopathic cystitis.

The compositions of the invention include cranberry fruit juice or extract, DL-methionine and at least one additional herb selected from the group consisting of *Ilex chinensis, Desmodium styracifolium*, or *Schisandra chinensis*. These herbs are used for their antiinflammatory, antimicrobial and stress-reducing properties. The resulting composition can be used as a dietary supplement or pharmaceutical formulation to address the risk factors associated with reoccurring urinary cystitis without introducing any harmful side effects. Thus, the present composition is useful as a therapeutic agent for the treatment of recurrent idiopathic cystitis or urinary tract infection and for maintaining the health of the urinary system.

It is thought that the cranberry fruit (*Vaccinium macrocarpon*) provides (i) acidification of urine, (ii) inhibition of bacterial adherence to bladder cells, and (iii) reduction of bacteriuria and pyuria. The cranberry fruit can be used in the form of fruit juice, fruit extract or dried fruit. When it is used in dried form it is preferably pulverized to a powder. When it is used in the form of an extract it is suitably an extract that can be obtained commercially, for example from Freeman Industries, L.L.C. 100 Marbledale Road, Tuckahow, NY10707-3420, which provides a 5:1 extract of cranberry fruit that has been standardized to contain about 3.4% quinic acid. Preferably, when employed in the herbal composition of the present invention the cranberry fruit can be provided as a 2:1 to about 10:1 extract which contains about 0.5% to about 10% quinic acid.

DL-Methionine is an amino acid which may help acidify urine and which can be obtained commercially. Preferably the DL-methionine used in the composition of the present invention is pharmaceutical grade containing 95% wt of DL-methionine.

In addition to cranberry fruit and DL-methionine, the present composition contains at least one Chinese herb. The herb is selected from *Ilex chinensis, Desmodium styracifolium* and *Schisandra chinensis*.

The herbs used in the herbal composition of the present invention may be employed in any suitable form, for instance as dried herb or as a herbal extract.

When dried Chinese herb is used it is preferably pulverized. In this embodiment the whole herb is dried and ground to a powder. The resulting powder of the or each herb is then conveniently mixed with powdered cranberry fruit or cranberry extract and powdered DL-methionine to form a herbal composition of the invention in powder form. This powder can be administered directly, for instance by being dispersed in a liquid for human subjects to drink or by being mixed into animal feed, especially cat feed, for animals to consume. Alternatively the powder can be processed into any other conventional dosage form such as tablets or granules.

When a herbal extract is used the extract is prepared by any conventional technique known for the extraction of ingredients from botanical material. These include solvent extraction and supercritical fluid extraction using a liquefied gas such as carbon dioxide.

If desired the extract may be dried before being formulated into a herbal composition of the invention, for instance by spray drying or by freeze drying (lyophilisation). In that case the dried Chinese herbal extract may be mixed with pulverized dried cranberry fruit or dried cranberry extract and powered DL-methionine to form a powder for direct administration to human or animal subjects, for instance as described above. Alternatively the extract may be used directly without prior drying.

The Chinese herbs or herb extracts are combined with the cranberry fruit and DL-methionine using any conventional technique that is suitable for ingredients of this type. When the Chinese herbs or herb extracts, the cranberry fruit and the DL-methionine are all in dry form they are conveniently mixed together, for instance by hand or by means of a mechanical mixer. A mixing procedure of this type may also be suitable if some, but not all, of the components of the herbal composition are in dry form.

When *Ilex chinensis* extract is employed it is preferably a pharmaceutical grade extract that can be obtained commercially, for example, from the Institute of Medicinal Plant Development, Haiding District, Xibeiwang, Beijing 100094, China, a Chinese manufacturer. Pharmaceutical grade *Ilex chinensis* extract manufactured in China is standardized for protocatechuic acid (>1.5 weight percent) and protocatechuic aldehyde (>0.15 weight percent). The pharmaceutical grade extract must pass extensive safety and efficacy procedures. Preferably, in accordance with the present invention the *Ilex chinensis* contains about 0.1 to about 10% weight percentage protocatechuic acid and about 0.01 to about 5% weight percentage protocatechuic aldehyde. More preferably, the *Ilex chinensis* contains at least 1.5 weight percent of protocatechuic acid and at least 0.15 weight percent of protocatechuic aldehyde. Without limiting the invention, *Ilex chinensis* may act as depicted in Figure 1 to reduce inflammation and inhibit the growth of microorganisms.

When *Desmodium styracifolium* extract is employed it is preferably a pharmaceutical grade extract that can be obtained commercially, for example from the Institute of Medicinal Plant Development, Haiding District, Xibeiwang, Beijing 100094, China, a Chinese manufacturer. The pharmaceutical grade extract must pass extensive safety and efficacy procedures. Pharmaceutical grade *Desmodium styracifolium* extract manufactured in China is standardized for total flavone content of about 6 weight percent. Preferably, when used in the practice of the present invention the *Desmodium styracifolium* extract has a flavone content of about 1 to about 25%. Preferably the minimum flavone content is at least 6 percent by weight, more preferably about 6 percent by weight. Without limiting the invention to a specific mechanism the action of the *Desmodium styracifolium* is thought to inhibit the formation of renal stones, decrease blood pressure and decrease inflammation as shown in Figure 1.

When *Schisandra chinensis* extract is employed it is preferably a pharmaceutical. grade extract that can be obtained commercially, for example, from a Chinese manufacturer, the Institute of Medicinal Plant Development, Haiding District, Xibeiwang, Beijing 100094, China. Pharmaceutical grade *Schisandra chinensis* extract manufactured in China is standardized for schisandrin and schisandrin B content of about 6.0 and 0.5 weight percent, respectively. The pharmaceutical grade extract must pass extensive safety and efficacy procedures. Preferably, when employed in the practice of the present invention the *Schisandra chinensis* extract has a minimum schisandrin content of about 1.0 to about 50% weight percentage, and a schisandrin B content of about 0.1 to about 20% weight percentage. Preferably, the *Schisandra chinensis* extract used in the present invention has a minimum schisandrin and schisandrin B content of at least about 6.0 and at least about *0.5* weight percent, respectively.

The herbal composition of the present invention as described above is typically formulated into a pharmaceutical or veterinary composition, or a dietary composition such as a dietary supplement, by conventional methods. In addition to the cranberry fruit, DL-methionine and Chinese herb or herbs the composition may include pharmaceutically, veterinarily or dietetically acceptable excipients as well as various additives. In one embodiment, the herbal composition of the present invention further comprises a vitamin, mineral, protein, fat or carbohydrate. The composition may comprise inert ingredients such as talc and magnesium stearate which are standard excipients in the manufacture of tablets, capsules and other dosage forms.

The pharmaceutically, veterinarily or dietetically acceptable excipient may be a solvent, dispersion medium, coating, isotonic or absorption delaying agent, sweetener or the like. These include any and all solvents, dispersion media, coatings, isotonic and absorption delaying agents, sweeteners and the like. These pharmaceutically acceptable carriers may be prepared from a wide range of materials including, but not limited to, diluents, binders and adhesives, lubricants, disintegrants, coloring agents, bulking agents, flavoring agents, sweetening agents and miscellaneous materials such as buffers and adsorbents that may be needed in order to prepare a particular dosage form. The use of such media and agents for pharmaceutically active substances is well known in the art. Except insofar as any conventional media or agent is incompatible with the active ingredient, its use in the present compositions is contemplated.

The present composition can be provided in any convenient form and for administration by any route such as orally, topically or parenterally. It can be provided as a dietary supplement or any pharmaceutical dosage form. It can be formulated into a food product or a drink, or in capsule form, tablet form, bar form, as a chewable gum or as a lotion. It can be provided, for example, as a snack bar, a cereal, a drink, a gum, or in any other easily ingested form. It can also be provided as a cream for topical application. One trained in the art can readily formulate the present composition into any of these convenient forms for oral or topical administration.

The herbal composition of the present invention is also suitably formulated into granules or a powder. In this form it can be readily dispersed in water or other liquid such as tea or a soft drink for human patients to drink. It can be equally readily mixed into feed for administration to animals such as cattle, cats and dogs.

According to the present invention, a therapeutically effective amount of the present compositions is an amount sufficient to relieve the symptoms of cystitis while minimizing harmful side effects. In one embodiment, the therapeutically effective amount is an amount sufficient to reduce the symptoms of recurrent idiopathic cystitis without causing harmful side effects. In another embodiment, the therapeutically effective amount is an amount sufficient to normalize urinary tract function without causing harmful side effects. Preferably, a daily dose (mg/kg-day) of the present dietary supplement or pharmaceutical formulation would be formulated to deliver per kg body weight of the mammal the following active ingredients within the suggested ranges: (a) about 10 to 40 mg/kg cranberry fruit extract; (b) about 5 to 50 mg/kg DL-methionine; (c) about 8 to 35 mg/kg *Ilex chinensis* extract; (d) about 8 to 35 mg/kg *Desmodium styracifolium;* and (e) about 1 to 7 mg/kg *Schisandra chinensis*. A preferred composition of the invention is formulated to deliver each component in the following amounts: (a) about 20 mg cranberry fruit extract per kg body weight; (b) about 40 mg DL-methionine per kg body weight; (c) about 21 mg *Ilex chinensis* extract per kg body weight; (d) about 18 mg *Desmodium styracifolium* per kg body weight; and (e) about 4 mg *Schisandra chinesis* per kg body weight. Dosages can be readily determined by one of ordinary skill in the art and can be readily formulated into the present supplemental and pharmaceutical compositions.

The invention will be further described in the Examples which follow:

### EXAMPLE 1: Preparation of herbal Composition

A herbal composition of the invention was prepared containing the following: DL-methionine, cranberry, *Ilex chineresis, Desmodium styracifolium* and *Schisartdra chinensis*.

Pharmaceutical grade DL-methionine was obtained commercially. Likewise the pharmaceutical grade cranberry extract (5:1) standardised to contain 3.4% quinic acid was obtained from Freeman Industries, L.L.C., USA. The other three herb extracts were specifically made at the Institute of Medicinal Plant Development, Beijing, China.

The authentication and quality evaluation of the raw herb material, critical to the production of the standardised herb extracts and the therapeutic effect of the finished product, were achieved by macroscopic and microscopic methods of inspection and authentication involving the application of expertise in pharmacognosy, phytochemstry and Chinese Materia Medica. In addition TLC and HPLC profiling were used.

The herb extracts of *Ilex chinensis, Desmodium styracifolium* and *Schisandra chinensis* were made separately with the extraction procedures designed specifically for each herb in order to achieve the expected therapeutic potency of the extracts. With the consistent concentration of 10:1 (1 g extract is equivalent to 10 g dry herb), each extract contained a set amount of selected chemical markers which were quantified by HPLC. The chemical marker content was 1.5% protocatechuic acid and 0.15% protocatechuic aldehyde in *Ilex chinensis* extract; 6.0% schisandra and 0.5% schisandra B in *Schisandra chinensis* extract and 6.0% total flavones in *Desmodium styracifolium* extract. Each extract was standardized.

The pharmaceutical grade cranberry extract and DL-methionine was combined by mixing with each of the Chinese herb extracts as described above to form a herbal composition.

### EXAMPLE 2: Preparation of Pharmaceutical Composition

A herbal composition prepared according to Example 1 was formulated into granules such that the following amounts of active ingredients would be supplied per kg body weight per day: (a) 20 mg/kg cranberry extract; (b) 40 mg/kg DL-methionine; (c) 21 mg/kg *Ilex chinensis* extract (1.5% wt protocatechuic acid and 0.15% wt protocatechuic aldehyde); (d) 6.5 mg/kg *Desmodium styracifolium* extract (6% wt total flavones); and (e) 4 mg/kg *Schisandra chinensis* extract (6.0% wt schisandrin and 0.5% wt schisandrin B).

### EXAMPLE 3: Clinical evaluation in the cat.

In a clinical study on cats, a tablet dosage form comprising the herbal composition described in Example 1 was administered daily to a group of cats observed to be suffering from symptoms of cystitis.

The tablets were ground into a powder and mixed into cat food for administration to each cat. Preliminary observations by a veterinarian indicated that there was an improvement in each animal's symptoms after two to five doses of the ground tablets.

## Claims

1. A herbal composition comprising cranberry fruit, DL-methionine and at least one Chinese herb selected from *Ilex chinensis, Desmodium styracifolium* and *Schisandra chinensis*.

2. A herbal composition according to claim 1 which comprises cranberry fruit, DL-methionine, *Ilex chinensis. Desmodium styracifolium* and *Schisandra chinensis*.

3. A herbal composition according to claim 1 or 2 comprising synergistically effective amounts of the cranberry fruit, DL-methionine and the or each said Chinese herb.

4. A herbal composition according to any one of the preceding claims wherein the cranberry fruit is in the form of cranberry fruit extract.

5. A herbal composition according to any one of the preceding claims formulated to deliver each component in the following ranges:
a. from 10 to 40 mg cranberry fruit extract per kg body weight;
b. from 5 to 50 mg DL-methionine per kg body weight;
c. from 8 to 35 mg *Ilex chinensis* extract per kg body weight;
d. from 2 to 25 mg *Desmodium styracifolium* per kg body weight; and
e. from 1 to 7 mg/kg *Schisandra chinensis* per kg body weight..

6. A herbal composition according to any one of the preceding claims formulated to deliver each component in the following amounts:
a. about 20 mg cranberry fruit extract per kg body weight;
b. about 40 mg DL-methionine per kg body weight;
c. about 21 mg *Ilex chinensis* extract per kg body weight;
d. about 18 mg *Desmodium styracifolium* per kg body weight; and
e. about 4 mg *Schisandra chinensis* per kg body weight

7. A herbal composition according to any one of the preceding claims wherein the cranberry fruit is in the form of 5:1 concentrate that has been standardized to contain 3.4% wt quinnic acid.

8. A herbal composition according to any one of the preceding claims
wherein the DL-methionine is pharmaceutical grade containing 95% wt of DL-methionine.

9. A herbal composition according to any one of the preceding claims wherein the *Ilex chinensis* contains at least about 1.5% wt protocatechuic acid and at least 0.15% wt protocatechuic aldehyde.

10. A herbal composition according to any one of the preceding claims wherein the *Desmodium styracifolium* contains a total flavone concentration of at least 6% by weight.

11. A herbal composition according to any one of the preceding claims wherein the *Schisandra chinensis* contains at least about 6% schisandrin and at least about 0.5% wt schisandrin B.

12. A herbal composition according to any one of the preceding claims which further comprises a vitamin, mineral, protein, fat or carbohydrate.

13. A dietary composition comprising a herbal composition as claimed in any one of the preceding claims and a dietetically acceptable excipient.

14. A composition according to claim 13 which is a dietary supplement.

15. A pharmaceutical or veterinary composition comprising a herbal composition as claimed in any one of claims 1 to 12 and a pharmaceutically or veterinarily acceptable excipient.

16. A composition according to any one of claims 1 to 15 which is formulated in capsule form, tablet form, bar form, as a chewable gum, as a lotion, or as a powder or granules.

17. A food product which includes a composition as claimed in any one of the claims 1 to 12.

18. Use of cranberry fruit, DL-methionine and at least one Chinese herb selected from *Ilex chinensis. Desmodium styracifolium* and *Schisandra chinensis* in the manufacture of a medicament for preventing or treating one or more symptoms of urinary cystitis.

19. Use according to claim 18 wherein the cranberry fruit, the DL-methionine and the or each said Chinese herb are used in synergistically effective amounts.

20. Use according to claim 18 or 19 wherein the said symptoms are selected from abnormal urinary function, irritative voiding, hematuria, inappropriate urination, urolithiasis and urinary tract infection.

## Patentansprüche

1. Pflanzliche Zubereitung, enthaltend Preiselbeeren ("cranberry fruit"), DL-Methionin und mindestens eine chinesische Kräuterart, die unter Ilex chinensis, Desmodium styracifolium und Schisandra chinensis ausgewählt ist.

2. Pflanzliche Zubereitung nach Anspruch 1, enthaltend Preiselbeeren, DL-Methionin, Ilex chinensis, Desmodium styracifolium und Schisandra chinensis.

3. Pflanzliche Zubereitung nach Anspruch 1 oder 2, enthaltend synergistisch wirksame Mengen an Preiselbeeren, DL-Methionin und eines oder mehrere der chinesischen Kräuter.

4. Pflanzliche Zubereitung nach einem der vorstehenden Ansprüche, wobei die Preiselbeeren in Form eines Preiselbeeren-Fruchtextrakts vorliegen.

5. Pflanzliche Zubereitung nach einem der vorstehenden Ansprüche, die so zubereitet ist, dass die einzelnen Komponenten in den folgenden Mengenbereichen abgegeben werden:
a. 10 bis 40 mg Preiselbeerenextrakt pro kg Körpergewicht;
b. 5 bis 50 mg DL-Methionin pro kg Körpergewicht;
c. 8 bis 35 mg Ilex chinensis-Extrakt pro kg Körpergewicht;
d. 2 bis 25 mg Desmodium styracifolium pro kg Körpergewicht; und
e. 1 bis 7 mg/kg Schisandra chinensis pro kg Körpergewicht.

6. Pflanzliche Zubereitung nach einem der vorstehenden Ansprüche, die so zubereitet ist, dass die einzelnen Komponenten in den folgenden Mengenbereichen abgegeben werden:
a. etwa 20 mg Preiselbeerenextrakt pro kg Körpergewicht;
b. etwa 40 mg DL-Methionin pro kg Körpergewicht;
c. etwa 21 mg Ilex chinensis-Extrakt pro kg Körpergewicht;
d. etwa 18 mg Desmodium styracifolium pro kg Körpergewicht; und
e. etwa 4 mg Schisandra chinensis pro kg Körpergewicht.

7. Pflanzliche Zubereitung nach einem der vorstehenden Ansprüche, wobei die Preiselbeeren in Form eines 5:1-Konzentrats vorliegen, das so standardisiert ist, dass es 3,4 Gew.-% Chinasäure enthält.

8. Pflanzliche Zubereitung nach einem der vorstehenden Ansprüche, wobei das DL-Methionin von pharmazeutischer Qualität ist und 95 Gew.-% DL-Methionin enthält.

9. Pflanzliche Zubereitung nach einem der vorstehenden Ansprüche, wobei Ilex chinensis mindestens etwa 1,5 Gew.-% Protocatechusäure und mindestens 0,15 Gew.-% Protocatechualdehyd enthält.

10. Pflanzliche Zubereitung nach einem der vorstehenden Ansprüche, wobei Desmodium styracifolium eine Konzentration an gesamten Flavonen von mindestens 6 Gew.-% enthält.

11. Pflanzliche Zubereitung nach einem der vorstehenden Ansprüche, wobei Schisandra chinensis mindestens etwa 6 % Schisandrin und mindestens etwa 0,5 Gew.-% Schisandrin B enthält.

12. Pflanzliche Zubereitung nach einem der vorstehenden Ansprüche, ferner enthaltend Vitamine, Mineralien, Protein, Fette oder Kohlenhydrate.

13. Diätetische Zubereitung, enthaltend eine pflanzliche Zubereitung nach einem der vorstehenden Ansprüche und ein diätetisch verträgliches Exzipiens.

14. Zubereitung nach Anspruch 13, bei der es sich um ein Nahrungsergänzungsmittel handelt.

15. Pharmazeutische oder veterinärmedizinische Zubereitung, enthaltend eine pflanzliche Zubereitung nach einem der Ansprüche 1 bis 12 und ein pharmazeutisch oder veterinärmedizinisch verträgliches Exzipiens.

16. Zubereitung nach einem der Ansprüche 1 bis 15, die in Kapselform, Tablettenform, Stäbchenform, als Kaugummi, Lotion, Pulver oder Granulat zubereitet ist.

17. Nahrungsmittelprodukt, enthaltend eine Zubereitung nach einem der Ansprüche 1 bis 12.

18. Verwendung von Preiselbeeren, DL-Methionin und mindestens einer der unter Ilex chinensis, Desmodium styracifolium und Schisandra chinensis ausgewählten chinesischen Kräuterarten bei der Herstellung eines Arzneimittels zur Prophylaxe oder Therapie von einem oder mehreren Symptomen von Blasenentzündung.

19. Verwendung von Anspruch 18, wobei die Preiselbeeren, das DL-Methionin und die chinesischen Kräuterarten in synergistisch wirksamen Mengen verwendet werden.

20. Verwendung nach Anspruch 18 oder 19, wobei die Symptome unter abnormaler Harnfunktion, irritativer Entleerung, Hämaturie, unangebrachtem Urinieren, Urolithiasis und Harnweginfektionen ausgewählt sind.

## Revendications

1. Composition contenant des herbes, qui comprend des fruits d'airelle, de la DL-méthionine et au moins une herbe chinoise choisie parmi *Ilex chinensis, Desmodium styracifolium et Schisandra chinensis*.

2. Composition contenant des herbes selon la revendication 1, qui comprend des fruits d'airelle, de la DL-méthionine, *Ilex chinensis, Desmodium styracifolium et Schisandra chinensis*.

3. Composition contenant des herbes selon la revendication 1 ou 2, qui comprend des quantités ayant une action synergique efficace, de fruits d'airelle, de DL-méthionine et de ladite herbe chinoise ou de chacune desdites herbes chinoises.

4. Composition contenant des herbes selon l'une quelconque des revendications précédentes, dans laquelle les fruits d'airelle sont sous la forme d'un extrait de fruit d'airelle.

5. Composition contenant des herbes selon l'une quelconque des revendications précédentes, qui est préparée de manière à fournir les divers constituants en les quantités suivantes :
a) 10 à 40 mg d'extrait de fruit d'airelle par kg de poids corporel,
b) 5 à 50 mg de DL-méthionine par kg de poids corporel,
c) 8 à 35 mg d'extrait d'*Ilex chinensis* par kg de poids corporel,
d) 2 à 25 mg de *Desmodium styracifolium* par kg de poids corporel, et
e) 1 à 7 mg de *Schisandra chinensis* par kg de poids corporel.

6. Composition contenant des herbes selon l'une quelconque des revendications précédentes, qui est préparée de manière à fournir les divers constituants en les quantités suivantes :
a) environ 20 mg d'extrait de fruit d'airelle par kg de poids corporel,
b) environ 40 mg de DL-méthionine par kg de poids corporel,
c) environ 21 mg d'extrait d'*Ilex chinensis* par kg de poids corporel,
d) environ 18 mg de *Desmodium styracifolium* par kg de poids corporel, et
e) environ 4 mg de *Schisandra chinensis* par kg de poids corporel.

7. Composition contenant des herbes selon l'une quelconque des revendications précédentes, dans laquelle les fruits d'airelle sont sous la forme d'un concentré 5:1, qui a été normalisé de manière à contenir 3,4 % en poids d'acide quinique.

8. Composition contenant des herbes selon l'une quelconque des revendications précédentes, dont la DL-méthionine est un produit de qualité pharmaceutique, contenant 95 % en poids de DL-méthionine.

9. Composition contenant des herbes selon l'une quelconque des revendications précédentes, dont l'*Ilex chinensis* contient au moins environ 1,5 % en poids d'acide protocatéchique et au moins 0,15 % en poids d'aldéhyde protocatéchique.

10. Composition contenant des herbes selon l'une quelconque des revendications précédentes, dont le *Desmodium styracifolium* contient une concentration totale de flavone d'au moins 6 % en poids.

11. Composition contenant des herbes selon l'une quelconque des revendications précédentes, dont le *Schisandra chinensis* contient au moins environ 6 % de schisandrine et au moins environ 0,5 % en poids de schisandrine B.

12. Composition contenant des herbes selon l'une quelconque des revendications précédentes, qui renferme en outre une vitamine, une matière minérale, une protéine, une matière grasse ou un hydrate de carbone.

13. Composition diététique qui comprend une composition contenant des herbes, telle que revendiquée dans l'une quelconque des revendications précédentes, et un excipient acceptable en diététique.

14. Composition selon la revendication 13, qui est un complément diététique.

15. Composition pharmaceutique ou vétérinaire, qui comprend une composition contenant des herbes, telle que revendiquée dans l'une quelconque des revendications 1 à 12, et un excipient acceptable en pharmacie ou dans l'art vétérinaire.

16. Composition selon l'une quelconque des revendications 1 à 15, qui se présente sous la forme de capsule, de comprimé, de barre, de gomme mastiquable, de lotion, de poudre ou de granules.

17. Produit alimentaire qui comprend une composition telle que revendiquée dans l'une quelconque des revendications 1 à 12.

18. Utilisation des fruits de l'airelle, de la DL-méthionine et d'au moins une herbe chinoise choisie parmi *Ilex chinensis, Desmodium styracifolium et Schisandra chinensis,* dans la préparation d'un médicament destiné à la prévention ou au traitement d'un ou plusieurs symptômes de la cystite urinaire.

19. Utilisation selon la revendication 18, dans laquelle les fruits de l'airelle, la DL-méthionine et ladite herbe chinoise ou chacune desdites herbes chinoises sont utilisés en des quantités ayant une action synergique efficace.

20. Utilisation selon la revendication 18 ou 19, dans laquelle lesdits symptômes sont choisis parmi la fonction urinaire anormale, l'évacuation irritative, l'hématurie, la miction inappropriée, la lithiase urinaire et l'infection des voies urinaires.
